# EUROPEAN PATENT SPECIFICATION

(11) **EP 4 048 331 B1**
(45) Date of publication and mention of the grant of the patent: **22.01.2025**
(21) Application number: 20829629.3
(22) Date of filing: 19.10.2020
(51) Int. Cl.: A61L 27/38, B29C 64/106, B29C 64/321, B29C 64/386, B33Y 50/00, C12N 5/00, B33Y 10/00, B33Y 70/00, B33Y 80/00, C12M 3/00

(54) **METHODS AND SYSTEMS FOR GENERATING THREE-DIMENSIONAL BIOLOGICAL STRUCTURES**
VERFAHREN UND SYSTEME ZUR ERZEUGUNG DREIDIMENSIONALER BIOLOGISCHER STRUKTUREN
PROCÉDÉS ET SYSTÈMES PERMETTANT DE GÉNÉRER DES STRUCTURES BIOLOGIQUES TRIDIMENSIONNELLES

(30) Priority: 21.10.2019 US 201962924111 P
(43) Date of publication of application: 31.08.2022
(73) Proprietor: Fluicell AB, 431 37 Mölndal (SE)
(72) Inventor: ORWAR, Owe, 43650 Hovås (SE); JEFFRIES, Gavin, D.M., 43146 Mölndal (SE); XU, Shijun, 42134 Göteborg (SE); KIREJEV, Vladimir, 43160 Mölndal (SE)
(74) Representative: Noréns Patentbyrå AB
(86) International application number: PCT/IB2020/000900
(87) International publication number: WO 2021/079192

(56) References cited:
- WO-A1-2019/021061
- SANGJUN MOON ET AL: "Layer by Layer Three-dimensional Tissue Epitaxy by Cell-Laden Hydrogel Droplets", TISSUE ENGINEERING PART C: METHODS, vol. 16, no. 1, 1 February 2010 (2010-02-01), pages 157 - 166, XP055180383, ISSN: 1937-3384, DOI: 10.1089/ten.tec.2009.0179
- MICHIYA MATSUSAKI ET AL: "Three-Dimensional Human Tissue Chips Fabricated by Rapid and Automatic Inkjet Cell Printing", ADVANCED HEALTHCARE MATERIALS, vol. 2, no. 4, 2 November 2012 (2012-11-02), DE, pages 534 - 539, XP055301992, ISSN: 2192-2640, DOI: 10.1002/adhm.201200299
- MICHIYA MATSUSAKI ET AL: "Fabrication of Cellular Multilayers with Nanometer-Sized Extracellular Matrix Films", ANGEWANDTE CHEMIE INTERNATIONAL EDITION, vol. 46, no. 25, 18 June 2007 (2007-06-18), pages 4689 - 4692, XP055382303, ISSN: 1433-7851, DOI: 10.1002/anie.200701089
- JEFFRIES GAVIN D. M. ET AL: "3D micro-organisation printing of mammalian cells to generate biological tissues", SCIENTIFIC REPORTS, vol. 10, no. 1, 10 November 2020 (2020-11-10), XP055786920, Retrieved from the Internet <URL:http://www.nature.com/articles/s41598-020-74191-w> DOI: 10.1038/s41598-020-74191-w

## Description

### CROSS-REFERENCE TO RELATED APPLICATION(S)

This application claims the benefit of priority of U.S. Provisional Patent Application Serial No. 62/924,111, filed October 21, 2019.

### BACKGROUND OF THE INVENTION

Additive manufacturing (also known as "3-D printing") has been proposed for various biological applications. Significant attention is for the improvement of preclinical models, where scientists in pharmaceutical development and clinical research are looking towards bioprinting technologies, to provide new *in vitro* tissue models with physiological relevant cell compositions, material properties and complex micro-structures. Bioprinting technology possesses the capability of precise positioning of biomaterials and living cells to reconstruct complex structures, with the goal of generating repeatable models of both diseased and healthy tissue. Bioprinting offers opportunities to integrate patient-specific cell sources into tissue models, further improving the biological relevance of *in vitro* screening.

WO 2019/021061 A1 discloses tissue-like structures being assembled using a microfluidic print head via cell deposition onto patterned substrates.

SANGJUN MON ET AL: "Layer by Layer Three-dimensional Tissue Epitaxy by Cell-Laden Hydrogel Droplets", TISSUE ENGINEERING PART C: METHODS, vol. 16, no. 1, 1 February 2010, pages 157-166 discloses a bioprinter that can be used to print multilayered 3D cell-laden hydrogel structures of tissue.

MICHIYA MATSUSAKI ET AL: "Tree-Dimensional Human Tissue Chips Fabricated by Rapid and Automatic Inkjet Cell Printing", ADVANCED HEALTHCARE MATERIALS, vol. 2, no. 4, 2 November 2012, pages 534-539 discloses layer-by-layer assembly using inkjet printing of single cells and proteins.

MICHIYA MATSUSAKI ET AL: "Fabrication of Cellular Multilayers with Nanometer-Sized Extracellular Matrix Films", ANGEWANDTE CHEMIE INTERNATIONAL EDITION, vol. 46, no. 25, 18 June 2007, pages 4689-4692 discloses multilayer cell films on a surface.

### SUMMARY OF THE INVENTION

One aspect of the invention provides a method of generating three-dimensional biological structures. The method includes: (a) depositing a first layer of a suspension over a substrate, the suspension including a liquid and a plurality of cells; (b) allowing the plurality of cells to attach to the substrate and form a first layer of attached cells; (c) depositing a cell-attachment agent over the first layer of attached cells; and (d) depositing a second layer of the suspension over the cell-attachment agent.

This aspect of the invention can have a variety of embodiments. The method can further include (b') culturing the plurality of cells in the first layer for a period of time in order to establish or increase the amount of, one or more selected from the group consisting of: cell-cell interactions, cell-cell attachments, cell-substrate interactions, cell-substrate attachments, cell mass, and cell quantity. The period of time can include one or more ranges of time selected from the group consisting of: from about 10 minutes to about 30 minutes, from about 30 minutes to about 60 minutes, from about 1 hour to about 12 hours, form about 12 hours to about 1 day, from about 1 day to about 3 days, from about 3 days to about 1 week, from about 1 week to about 2 weeks, from about 2 weeks to about 4 weeks, and from about 4 weeks to about 8 weeks.

The method can further include: (e) repeating steps (b)-(d) until a biological structure having a predefined thickness is generated. The predefined thickness can be selected from the group consisting of: about 1 µm, between about 1 µm and about 10 µm, between about 10 µm and about 100 µm, between about 100 µm and about 1 mm, between about 1 mm and about 1.5 mm, between about 1.5 mm and about 5 mm, between about 5 mm and about 2 cm, between about 2 cm and 10 cm, and between about 10 cm and 50 cm .

The method further includes: (e) repeating steps (a)-(c) at a new location on the substrate.

The suspension can be deposited using a flow-confinement device. The cell-attachment agent can be applied globally or in a pattern. The suspension can be applied globally or in a pattern.

The suspension can include a single cell type or a plurality of cell types.

The substrate can be selected from the group consisting of: tissue and organ.

The method can further include: (f) depositing a cell blocking agent over the second layer of the suspension. The cell-blocking agent can be selected from the group consisting of: polyethylene glycol (PEG), block copolymers, bovine serum albumin (BSA), surfactant, casein, polytetrafluoroethylene (PTFE), silanes, particulate solutions, nanoparticles, metal nanoparticles, gold nanoparticles, silver nanoparticles, aluminum nanoparticles, titanium nanoparticles, and polymeric microspheres.

The cell-attachment agent can be selected from the group consisting of: adhesion proteins, cell-attachment proteins, cell-adhesion biomolecules, cell-adhesion molecules, polylysine, laminin, fibronectin, vitronectin, extracellular matrix (ECM), actin, integrin, perlecan, desmin, poly glycans, fibulin, elastin, tropoelastin, cadherin, gelatin, concanavalin, collagen, lipid structures, lipid nanotubes, lipid vesicles, lipid droplets, growth serum, fetal bovine serum, calf serum, horse serum, nucleic acids, DNA strands, RNA strands, cationic solutions, calcium solutions, magnesium solutions, and zinc solutions.

The substrate can be selected from the group consisting of: cell-attachment agent, plastic, glass, silicon, paper, cotton, biomaterial, lipid films, gel, aerogel, hydrogel, polymer matrix, metals, metal oxides, semipermeable membranes, permeable membranes, polysaccharides, starch, collagen, cellulose, gelatin, biological scaffolds, biological tissue, decellularized tissue, non-biological tissue, and biological cells.

The substrate can have a planar, uneven, structured, or patterned topology.

The substrate can include adjacent sections of different substrate materials.

### BRIEF DESCRIPTION OF THE DRAWINGS

For a fuller understanding of the nature and desired objects of the present invention, reference is made to the following detailed description taken in conjunction with the accompanying drawing figures wherein like reference characters denote corresponding parts throughout the several views.

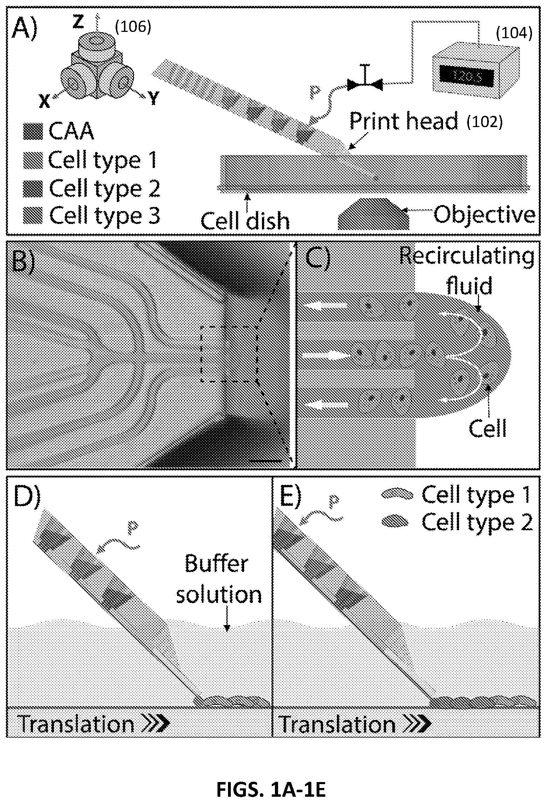

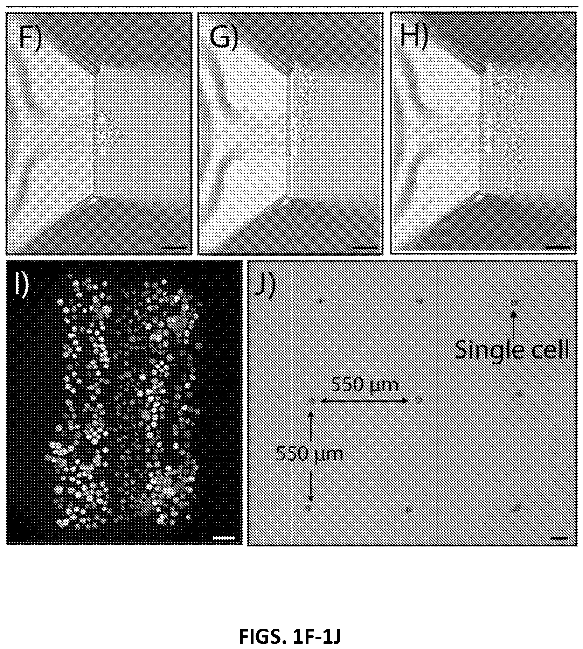

FIGS. 1A-1J depict an exemplary direct cell bioprinting approach, highlighting exemplary components. FIGS. 1A-1C illustrate printing cells using a recirculating fluid flow. Specifically, FIG. 1A provides a schematic overview of the bioprinting setup consisting of; a micro-positioner-controlled printhead, an automated substrate positioner, a computer-controlled pneumatic interface, and a microscopy imaging system. FIG. 1B provides an exemplary fluorescent microscopy image of the fluidic printhead tip, demonstrating the hydrodynamically confined flow. FIG. 1C provides an illustration of the fluidic printhead exit, presenting the flow path of cells within the confined circulating fluid. FIGS. 1D-1E illustrate an exemplary method of pattern formation using multiple cell types. FIG 1D demonstrates that positioning of the printhead in proximity to a substrate surface within a buffer solution, while maintaining a confined fluid flow, enables cells to interact and attach to the substrate within a localized region at the tip of the printhead. Translating the surface relative to the printhead allows the deposited cells to be patterned on said surface, *e.g.,* to modulate fluid flow over the resulting substrate. FIG. 1E demonstrates that individual solutions containing, *e.g.,* different cells from individual sample wells within the printhead can be changed at will, allowing for numerous loaded cell types to be printed without the need to replace or reload the printhead. FIGS. 1F-1H depict experimental images demonstrating this approach, by printing a structured pattern composed of alternating printed stripes of HaCaT and A431 cells onto a petri dish surface immersed in DMEM growth media. FIG. 1F depicts A431 cells positioned within the confined flow at the beginning of a structured print. FIG. 1G depicts the first stripe of the pattern being printed by translating the substrate. FIG. 1H depicts an image of the pattern mid-way through printing the second cell stripe, after switching cell type to HaCaT. The final printed structure is shown in FIG. 1I, composed of alternating printed stripes of HaCaT and A431 cells, labelled with cytotracker-green and cytotracker-red respectively. This structure was post-print nuclei stained with HOECHST^{®} 33342 and fixed with paraformaldehyde. A demonstration of the printing precision is shown in FIG. 1J, whereby a single cell array was constructed using SH-SY5Y cells. Through automated control of the substrate positioning, the spacing between the cells can be fixed or adjusted in real-time. The scale bar in FIGS. 1F to 1J represents 100µm.

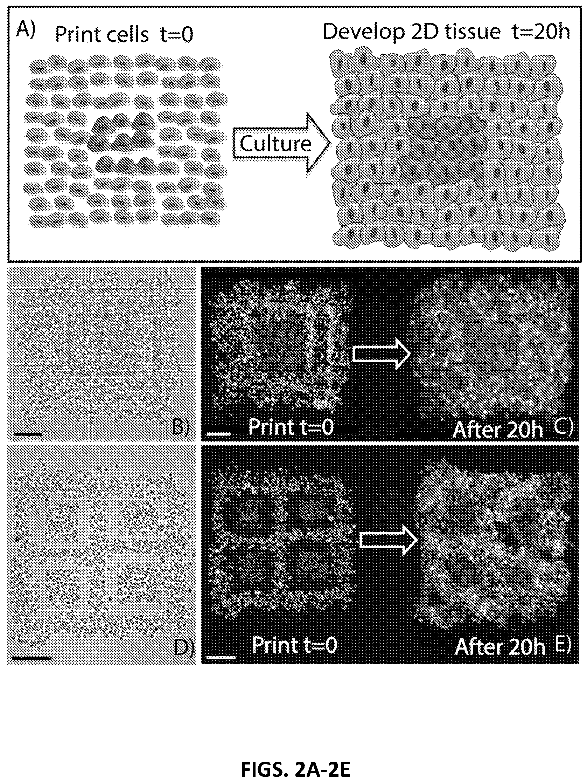

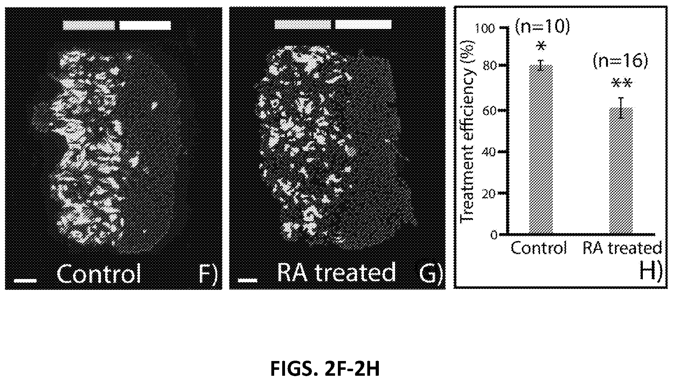

FIGS. 2A-2H depict exemplary post-printing tissue and cell colony model development as well as cell growth. Complex tissue and cell colony models using, *e.g.,* small samples containing few cells can be built by incubating the printed samples, allowing the cells to grow and form cell-to-cell interactions resulting in a tissue or a continuous weave of cells. A pictographic representation of the model development is shown in FIG. 2A as the freshly deposited cells grow and proliferate to establish a tissue-like structure. FIGS. 2B-2E present experimental examples, highlighting before and after a 20-hour incubation post-printing. A simple encapsulated patch of skin cancer cells (A431) surrounded by, and in contact with, an epithelial cell layer (HaCaT) is shown in FIGS. 2B-2C whereby HaCaT and A431 cells were labelled with cytotracker-green (green dashed boundary) and cytotracker-red (red dashed boundary) respectively. A brightfield image of the pattern directly after printing is shown in FIG. 2B, where dashed lines indicate the boundaries of the printed cell types. The corresponding fluorescent image is shown in the left panel of FIG. 2C, whereby the grown model is presented in the right panel of FIG. 2C. A more complex version of this encapsulation model is presented in FIGS. 2D and 2E, whereby four skin cancer cell patches (A431) are printed and surrounded by an epithelial cell layer (HaCaT). The two cell types were printed to not be in contact, to allow each cell-type to attach to the surface and grow prior to interacting with each other. FIGS. 2D and 2E were performed using the same timing and conditions as for FIGS. 2B and 2C. A simple application of this approach was achieved by establishing a psoriasis-like printed skin model. The skin model responded to retinoic acid-a drug used in psoriasis therapy. This model is composed of a two-printed stripe structure, of differentiated and non-differentiated HaCaT cells. The cell stripes were printed and allowed to proliferate for 24 hours prior to being tested. Control samples were exposed to fresh growth media and treated samples were exposed to retinoic acid supplemented media for 20 hours. Following the treatment, all samples were fixed with acetone and labelled with HOECHST^{®} 33342 (blue) to identify the nuclei and a double antibody staining, first with rabbit anti-human cytokeratin 10, followed by goat anti-rabbit IgG ALEXA FLUOR^{®} 488 (green) to identify the level of CK10 expression. Example images of fixed treated samples are shown in FIGS. 2F and 2G, where green and white bands at the top of the panels indicate the approximate printed stripe width of differentiated and non-differentiated cells respectively. A summary plot of the retinoic acid treatment is shown in FIG. 2H, indicating an approximately 20% decrease in CK10 expression for the treated samples. The scale bar in FIGS. 2B-2G represents 200 µm.

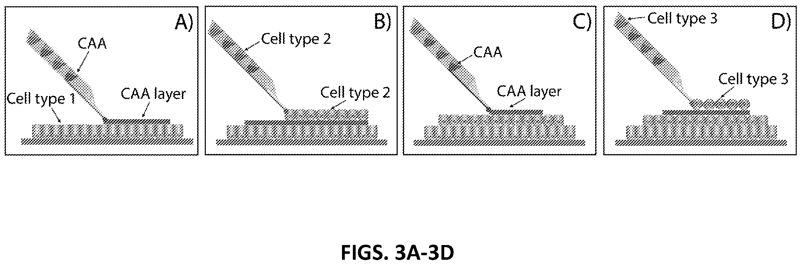

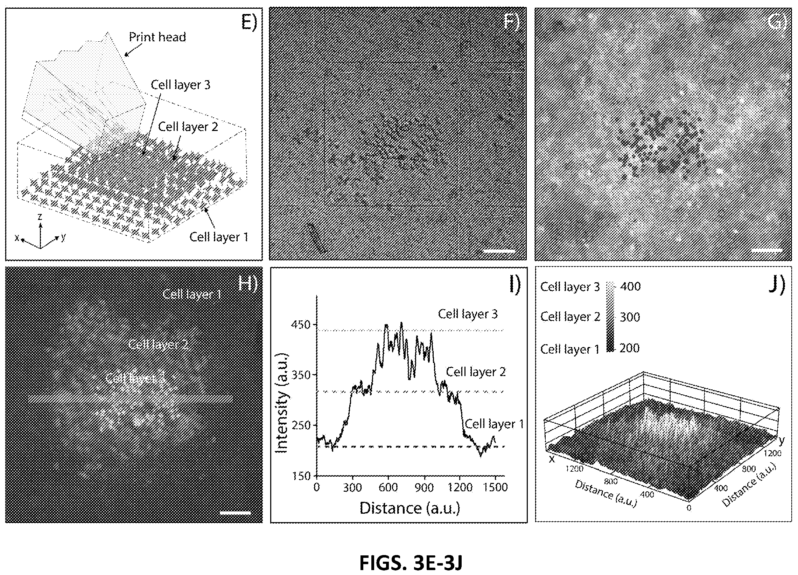

FIGS. 3A-3J demonstrate 3D cell bioprinting by applying a direct cell bioprinting approach to establish a multi-layered, 3D multi cell-type structure. An overview of the printing scheme is presented in FIGS. 3A to 3D, wherein a substrate surface is pre-cultured with a confluent cell layer, upon which a layer of a cell attachment agent (CAA) applied with the bioprinter head can then be deposited, as shown in FIG. 3A. Directly after CAA deposition, a second layer of cells can be printed, as shown in FIG. 3B. This multi-layered structure is then incubated and allowed to grow for 24 hours. This process of depositing CAA, followed by printing cells and incubating, can be cycled or repeated several times to establish additional layers, as shown in FIGS. 3C and 3D, resulting in a 3D patterned structure of cells, shown in FIG. 3E. An experimental example of this 3D layering approach is shown in FIGS. 3F to 3H and is demonstrated to build a patterned structure of A431 cells, followed by HaCaT cells, followed by A431 cells. The printing substrate was a coated 500µm gridded petri dish, to aid in identification and printing of each subsequent layer. A brightfield image of the final 3D printed structure, directly after printing the third layer, is shown in FIG. 3F. The base cell layer being composed of A431 cells labelled with cytotracker-green (green), the middle layer being HaCaT cells labelled with cytotracker-red (red), and the top layer being A431 cells pre-printing stained with Hoechst 33342 (blue). A fluorescence overlay of the three color channels is shown in FIG. 3G. As a visualization aid, each channel was normalized to an 8-bit greyscale image then summed together as a 32-bit image, to readily establish where cells are layered on top of each other, shown in FIG. 3H. A line profile was taken through the field shown in FIG. 3H, corresponding to the orange line. A plot was generated shown in FIG. 3i, which indicates the heights of fluorescence intensity after image reconstruction. An alternate representation of this layered visualization is shown in FIG. 3J, highlighting the final multi-layered print layout. The scale bar in FIGS. 3F to 3J represents 100µm.

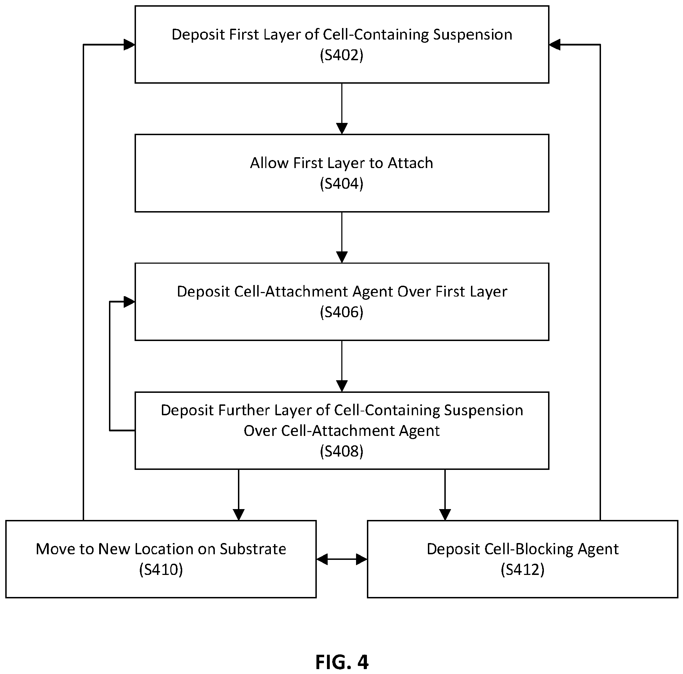

FIG. 4 depicts an exemplary method of generating three-dimensional biological structures.

### DEFINITIONS

The instant invention is most clearly understood with reference to the following definitions.

As used herein, the singular form "a," "an," and "the" include plural references unless the context clearly dictates otherwise.

Unless specifically stated or obvious from context, as used herein, the term "about" is understood as within a range of normal tolerance in the art, for example within 2 standard deviations of the mean. "About" can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

As used herein, the term "attachment" can refer to both physical, chemical, biological ionic, and electrostatic interactions, such as steric, covalent, ionic and hydrogen bonds, between cells and a substrate, surface or other cells. Attachment can be formed on the order of a minute for some cells. Other cells (e.g., neurons) may attach over weeks. Exemplary time ranges for allowing for attachment may include: from about 1 second to about 10 seconds, from about 10 seconds to about 30 seconds, from about 30 seconds to about 60 seconds, from about 1 minute to about 10 minutes, from about 10 minutes to about 30 minutes, from about 30 minutes to about 60 minutes, from about 1 hour to about 12 hours, form about 12 hours to about 1 day, from about 1 day to about 3 days, from about 3 days to about 1 week, from about 1 week to about 2 weeks, from about 2 weeks to about 4 weeks, and from about 4 weeks to about 8 weeks, from about 8 weeks to about 12 weeks, and so on.

As used in the specification and claims, the terms "comprises," "comprising," "containing," "having," and the like can have the meaning ascribed to them in U.S. patent law and can mean "includes," "including," and the like.

Unless specifically stated or obvious from context, the term "or," as used herein, is understood to be inclusive.

Unless specifically stated or obvious from context, the term "recirculation" can have the meaning of a fluid flow path exiting the device and returning to the same device.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 (as well as fractions thereof unless the context clearly dictates otherwise).

### DETAILED DESCRIPTION OF THE INVENTION

Aspects of the invention provide systems and methods for the controlled deposition of biological cells or cell constituents onto surfaces, including but not limited to the additive manufacturing of small-to-large cellular networks, tissues, organs, and other biological structures. In particular, embodiments of the invention can be used to create histologically complex cell structures in 2D and 3D.

### Fluid Dispensers

In some embodiments of the invention, the fluid dispenser includes a nozzle or other device that ejects a fluid. For example, embodiments of the invention can be applied to existing additive manufacturing (also known as "3-D printing") devices, e.g., by incorporating the systems and methods described herein alongside existing additive manufacturing printheads that apply materials such as polymers, *e.g.,* biocompatible polymers, for the production of organs-on-a-chip.

Referring now to FIGS. 1A-1H, in some embodiments, the printing systems and methods utilize a flow-confinement device 102 and a controller 104 configured to generate a confined liquid volume outside of the flow-confinement device 102 where the confined liquid volume can be modulated by said controller 104 to include modulated and non-confined flow modes, as well as zero-flow modes, and where the controller 104 can be used to switch between any flow modes in arbitrary sequence, for arbitrary periods of time.

Some embodiments of the invention utilize fluid flows or recirculating fluid flows, generated at the tip of a flow-confinement device 102, to achieve high resolution printing (*e.g.,* with respect to both the lateral and axial position of the cells, deposition spot size for cell groupings, layer thicknesses, quantities of cells deposited, types of cells deposited, and the like). Although embodiments of the invention provide single-cell-deposition capabilities, embodiments of the invention can also deposit multiple cells at a time. For example, and without being bound by theory, Applicant believes that embodiments of the invention can reliably deposit layers having a thickness of less than two cells (*e.g.,* less than about 500 µm, less than about 300 µm, less than about 200 µm, less than about 150 µm, less than about 100 µm, less than about 75 µm, less than about 50 µm, less than about 20 µm, less than about 10 µm, less than about 5 µm, and the like).

As depicted most clearly in FIG. 1C, flow-confinement device 102 can include one or more channels of which some channels are configured as outlet channels, through which liquid leaves the device 102 into an open volume and some channels are inlet channels, through which liquid is withdrawn from the open volume into the device. In some embodiments, outlet channels and inlet channels are different channels. In some embodiments same channel can be intermittently switched to be outlet channel and an inlet channel, *e.g.,* as further described in U.S. Patent No. 9,126,197.

Controller 104 can be configured to control the liquid flow such that all liquid leaving from the device is returning eventually back into the device when in recirculatory mode. Outflow can reach a limited maximal distance from the outlet channel before returning into the inlet channel. This distance defines the size of the confined liquid volume. Therefore, the liquid outflow can reach a substrate (*e.g.,* cell dish in FIG. 1A) or a surface, if it is positioned closer than the critical dimension of the confined liquid volume. In some embodiments, the outflow can reach the substrate, such that the flow-confinement device 102 does not have a direct contact with the substrate. The tip of the flow-confinement device 102 can, in some non-limiting applications, be in contact with the substrate.

In various embodiments, the device can reliably deposit single cells, and or groups of cells, with a lateral resolution on the order of a single cell (*e.g.,* less than about 150 µm, less than about 100 µm, less than about 75 µm, less than about 50 µm, less than about 30 µm, less than about 20 µm, and the like).

Exemplary devices and methods for generating confined fluid flows are described in U.S. Patent Nos. 9,126,197; 9,658,240; and 9,671,366.

### Exemplary Control Systems

Still referring to FIG. 1A, the recirculating fluid flow-confinement device 102 can be readily positioned relative to an object of interest using either a manual positioning system, or through the implementation of an electronically controlled positioning device 106. The positioning system may include hydraulic, mechanical, or electrical (*e.g.,* stepper or servo motor) micromanipulators. These actuators can be arranged in one, two, three, four or greater directions.

The electronically controlled position of both the printhead and the substrate can be achieved through on-demand control, *e.g.,* through the use of a joystick, gamepad controller, scroll wheel, a touch screen and combinations thereof, and/or through the use of a computationally determined path. These paths can for example, be derived from a simple coordinate system, a design file, such as .stl (STereoLithography) CAD files, and/or through a relative feedback response, *e.g.,* adapting to the changing position of cell propagation.

### Feedback

In some embodiments, methods of cell deposition may be carried out under the control of a preprogrammed flow controller. In various embodiments, the system may further comprise a cell concentration measuring device for measuring cell deposition. In various embodiments, the preprogrammed flow controller may be programmed to assess whether a predefined number of cells are correctly located on the substrate. The feature of various embodiments by which parameters of the method are adjusted based on information collected over the course of the method is termed "feedback".

In various embodiments, the system may further comprise a device or method for measuring a cell property, such as a method for measuring protein expression, gene expression, metabolic markers, cell-cell interactions, ions, or other biological, physical or chemical properties of one or several cells in the various flow modes, including recirculatory flow.

The concentration of particles/cell in the flow can be determined from either the initial concentration as it entered the microfluidic device and/or monitoring within the dispenser or on the substrate, e.g., conductivity, impedance, fluorescence, phase retardation and the like.

Coverage can be assessed through a variety of techniques and devices. By way of non-limiting example, coverage may be assessed by optical assessment, particle tracking, by measuring conductivity and/or impedance, by ultrasound and/or by optical phase retardation measurements. A person of skill in the art will appreciate that different techniques for measuring coverage are more or less appropriate based on circumstances including, for example, the cell type and the type of liquid or liquids used.

In one embodiment, optical assessment can be performed using a digital camera (*e.g.,* a charge-coupled device (CCD) or complementary metal oxide semiconductor (CMOS) image sensor), which can optionally be associated with various optics such as microscope, a light source, and the like. Suitable imaging modalities such as reflection, absorption, phase retardation, polarization, fluorescence, phosphorescence, and the like. Image processing can be performed on the captured image and examined to determine if the current coverage state is acceptable (*e.g.,* in view of a .stl source file). Various patterns can place the same or similar cells adjacent to each other, place different cells adjacent to each other, place cells adjacent to non-cellular materials such as polymeric scaffolds, and the like.

In another embodiment, suspended particles are tracked, *e.g.,* after exiting from the microfluidic printhead. For example, sperm tracking technology such as described in U.S. Patent Application Publication No. 2017/0109879 can be applied to track a plurality of moving suspended particles. The fluid flow can be modified or paused in order to facilitate desired placement of the suspended particle(s).

In another embodiment, conductivity and/or impedance can be utilized to assess coverage. For example, two or more electrodes can be positioned in the deposition chamber, at least one of which should be adjacent to the microfluidic printhead, and current can be applied between the electrodes. Without being bound by theory, Applicant believes that resistance will increase in measurements during recirculating fluid flow as cells are deposited. Likewise and without being bound by theory, Applicant believes that the presence of deposited cells after cessation of the recirculating fluid flow will result in decreased impedance. Result-indicative conductivity and/or impedance values can be determined experimentally for particular embodiments, stored in computer-readable media, and referenced in a particular embodiment.

In still another embodiment, acoustic waves (*e.g.,* ultrasound waves) can be utilized to detect the presence and position of a recirculating fluid flow and/or a deposited particle.

In yet another embodiment, deposited particle position and/or surface coverage may be determined by optical phase retardation measurements. Any source of polarized light and as well as any technique for phase detection, such as digital holographic microscopy (DHM) may be employed.

### Exemplary Substrates

As used herein, the term "substrate" refers to the area or the object in or on which cells are deposited. The term "substrate" is to be construed broadly as the methods of the invention may be applied to a wide variety of different substrates. By way of non-limiting example, the substrate may be a surface, such as glass or plastic, which may or may not be functionalized. The substrate may be a volume element, such as a contained fluid or a gel. The substrate may also be cells of the same type or different from those deposited. In embodiments in which the substrate is a cell or cells, the cells may be suspended in a solution, attached to a surface or another object, or part of tissue, either in culture or from a living organism including a human.

The substrate can, among other materials, be composed of: a cell-attachment agent, plastic, glass, silicon, paper, cotton, biomaterial, lipid films, gel, aerogel, hydrogel, polymer matrix, metals, metal oxides, semipermeable membranes, permeable membranes, polysaccharides, starch, cellulose, gelatin, biological scaffolds, biological tissue, non-biological tissue, biological cells, or a composite material of any of the preceding list.

Exemplary plastic types include, but are not limited to, polycarbonate, polystyrene, polyethylene terephthalate, high-density polyethylene, low-density polyethylene, polyvinyl chloride, polypropylene, polystyrene, polylactide, polyacetal, poly dimethyl siloxane, acrylic, acrylonitrile butadiene, nylon, and the like.

### Exemplary Suspensions

The suspensions dispensed by the printhead can include a variety of solvents including aqueous solvents. The viscosity of the suspension can be configured so that the suspension remains in a desired location for sufficient time for cell attachment and/or growth. In some embodiments, the solvent is a dilute gel.

### Exemplary Biological Materials

The dispensed suspensions can include a variety of biological materials such as cells, cell fragments, vesicles, subcellular organelles, particles, and the like.

Cell suspensions may be prepared using standard techniques, as understood in the art. For example, mammalian cells may be suspended in a solution of about 1×10⁶ cells/mL to about 10×0⁶ cells/mL. The cell suspension solution may be combined with a PEG (*e.g.,* mol wt 6000). In some embodiments, the cell suspension solutions may have a final concentration of about 15 mg/ml.

In some embodiments, each layer includes a single cell type. In other embodiments, a plurality of cell types are provided in the same suspension. In still other embodiments, a plurality of suspensions, each containing a single cell type are applied adjacent to each other within a single layer. In yet another embodiment, the cell types vary from layer-to-layer.

### Exemplary Cell Attachment Agents

The cell attachment agent (CAA) can be delivered globally (over the whole sample surface), locally (within a defined region of the sample) and/or directly patterned (over the whole sample or locally, where the region is defined by the path of the fluidic printhead).

CAA types include, but are not limited to adhesion proteins, cell-attachment proteins, cell-adhesion biomolecules, cell-adhesion molecules and further include, but are not limited to: polylysine (*e.g.,* poly-L-lysine (PLL)), laminin, fibronectin, vitronectin, extracellur matrix (ECM) (*e.g.,* available under the GELTREX^{®} mark from Life Technologies Corporation of Carlsbad, California), actin, integrin, perlecan, desmin, poly glycans, fibulin, elastin, tropoelastin, cadherin, , gelatin, concanavalin, collagen, organic and inorganic gels, lipid structures (*e.g.,* nanotubes, vesicles, droplets, and the like), growth serum (*e.g.,* fetal bovine serum, calf serum, horse serum, and the like), nucleic acids (*e.g.,* DNA and/or RNA strands), cationic solutions (containing, *e.g.,* calcium, magnesium, zinc, and the like), or a composite solution comprised of any of the preceding list.

The CAA may be prepared as a working solution for pretreating one or more substrates. The CAA working solution for pretreating may include one or more of PLL, poly-D-lysine (PDL), ECM, and a buffer including for example sodium borate buffer. In certain embodiments, the working solution for pretreating includes: 0.5 mg/mL poly-L-lysine (PLL) and 75 µg/mL extracellular matrix (protein concentration 120-180 µg/mL, GELTREX^{®}, Gibco), prepared using 0.1 M sodium borate buffer. The working solution for pretreating may be prepared a time interval before use, including immediately prior to use.

The CAA may be prepared as a working solution for direct printing, further known as CAA-dp. The CAA-dp may be of the same composition as CAA or be of a composition including one or more of PLL, PDL, serum (*e.g.,* fetal bovine serum), and buffer (*e.g.,* phosphate-buffered saline buffer). In certain embodiments, the working solution for direct printing includes: 0.1 mg/mL poly-L-lysine (PLL) and 10% FBS, prepared in a 1× phosphate-buffered saline (PBS) buffer. The working solution for direct printing may be prepared a time interval before use, including immediately prior to use.

CAA may additionally include envrinmentally responsive compounds, such as Poly(N-isopropylacrylamide (PNIPAAm) for thermal responsivity or Rose Bengal or riboflavin for photoactive linkers.

### Exemplary Cell-Blocking Agents

In a similar manner to CAA, cell-blocking agent (CBA) can be applied globally over a substrate, locally, or patterned using the fluidic printhead. The CBA can prevent attachment between adjacent cells and/or act as a placeholder. In some embodiments, the CBA is a dissolvable substance. For example, a CBA such as PEG can be deposited as a scaffold to establish a lumen while cells are deposited around the lumen to form a vessel.

CBA types include, but are not limited to, polyethylene glycol (PEG), block copolymers, bovine serum albumin (BSA), surfactants (*e.g.,* PLUORONIC^{™} surfactants available from ThermoFisher Scientific of Waltham, Massachusetts), casein, polytetrafluoroethylene (PTFE) (*e.g.,* TELLON^{®} available from The Chemours Company of Wilmington, Delaware), silanes, particulate solutions (*e.g.,* including nanoparticles (*e.g.,* metal nanoparticles such as gold, silver, aluminium, titanium, and the like), polymeric microspheres, and the like), or a composite solution comprised of any of the preceding list.

### Additions to Living Organisms

Embodiments of the invention can be applied to add material to living organisms. In such embodiments, the organism can be considered the substrate. Such embodiments can be used for a variety of therapeutic and/or cosmetic applications including regenerative medicine, tissue grafting, plastic surgery, orthopedics, and the like.

Embodiments of the invention utilizing a soft printhead are particularly advantageous in such embodiments, especially when applied to delicate tissues such as the eye and the ear.

Such an *in*-*situ*-generated graft can be bound to the organism through various techniques including the use of cross-linking as discussed herein.

*In-situ*-generated grafts can also be used for research purposes. For example, cancer cells can be printed within an organism (*e.g.,* in a predefined and consistent pattern across multiple organisms) for experimental animal research.

The references to methods of treatment above, under heading "Additions to Living Organisms", are to be interpreted as references to the compounds, pharmaceutical compositions and medicaments of the present invention for use in a method for treatment of the human (or animal) body by therapy (or for diagnosis).

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

### Example 1: Preparation of a Printed Skin Model

In order to prepare an exemplary three-dimensional skin model, the following materials were prepared and used according to the following methods.

### Materials and Methods

### Cell Culture

HaCaT cells were maintained and grown in complete Dulbecco's Modified eagle's Medium (DMEM, Gibco) supplemented with 10% fetal bovine serum (FBS, Gibco), at 37°C and 5% CO₂. Cells used as experimental controls were sub-cultured when they attained a confluency of approximately 80%, preventing the cells from differentiating. To mimic psoriasis-affected cells, a separate culture of HaCaT cells were prepared and allowed to differentiate, by growing the culture for 3-4 additional days, once a 100% confluency had been achieved.

### Cytokine Treatment

To induce the psoriasis-like protein expression, differentiated HaCaT cells were exposed to a combination of inflammatory cytokines IL-1α (10 ng/ml, Peprotech) and TNF-α (5 ng/ml, Peprotech) for 36 hrs prior the bioprinting process.

### Bioprinting

Undifferentiated and differentiated psoriasis-like (cytokine-treated) HaCaT cells were used for bioprinting a skin model with the BIOPIXLAR^{®} platform. The skin model was formed by printing stripes of psoriasis-like ("diseased") and undifferentiated ("healthy") cells side-by-side. The printing process was performed on CAA-precoated IBIDI^{®} dishes. After printing, the cells were allowed to grow and proliferate in full growth media (DMEM +10% FBS) at 37°C, 5% CO₂ for 24 hrs.

In general, the following protocol was utilized for printing the skin model as described herein.
1. Preload the printhead with the required cell suspensions and other reagents. If the substrate surface is not pre-treated with CAA, CAA-dp solution needs to be additionally preloaded into the printhead.
2. Add suitable amount of cell culture medium without FBS onto the substrate, to establish immersion for the printhead, then position the printhead relative to surface.
3. If the substrate has not been CAA coated, the target print area can be directly coated with CAA-dp prior to printing the cells.
4. Cells are printed onto the substrate as desired.
5. After cell printing, the substrate is transferred to an incubator (37°C and 5% CO₂) and allowing the cells to attach, proliferate and/or grow until the desired cell layer is achieved. FBS can be added to the growth media after printing.
6. To build additional cell layers, the whole substrate**, including the grown cells, can be pre-treated with CAA-dp by replacing the growth medium with CAA-dp and incubating (37°C and 5% CO₂) for 5 min. This solution can then be removed, and the cell-laden substrate gently rinsed with 1× PBS buffer or culture medium without FBS.
7. Prior to printing, the additional layer of cells, the solution in which the substrate is immersed can be exchanged with culture medium without FBS.
8. Repeat step 3-7 for printing a new cell layer.
** If a localized area is to be treated for printing instead of the global substrate, the CAA-dp can be locally applied by exposing the surface to the CAA-dp solution using the printhead. This localized patterning does not require a complete change of the substrate immersion medium.

### Drug Treatment

To verify the applicability of the bioprinted skin model, the effect of all-trans retinoic acid (RA) (Sigma), a drug used for many skin disorders, was compared against a control. Four hours post-printing, RA was introduced to the full growth media to a final concentration of 1 µM. The model was then allowed to grow for 20 hrs. The control model was treated with equal amounts of the RA solvent.

### Immunofluorescence Staining

Culture media was removed from the dish and the printed models were washed three times with prewarmed (37°C) PBS. The cells were then fixed with chilled acetone for 5-6 minutes at -20°C. Acetone was removed from the dish then thoroughly washed with PBS without allowing the cells to dry between rinses. Thereafter, fixed cells were incubated with ULTRACRUZ^{®} Blocking reagent (Santa Cruz Biotechnology, USA) for 30 minutes at room temperature (RT). After blocking, cells were stained with rabbit primary antibody (Santa Cruz Biotechnology, USA) diluted in ULTRACRUZ^{®} Blocking reagent at RT for 1 hr. Next, cells were washed three times with PBS and stained with anti-rabbit secondary antibody conjugated with ALEXA-FLUOR^{®} 488 (Santa Cruz Biotechnology, USA) at RT for 1 hr. Afterwards, cells were washed again three times with PBS, and mounted using ULTRACRUZ^{®} Aqueous Mounting Medium with DAPI (Santa Cruz Biotechnology, USA), and sealed with coverslip. Images were taken using a fluorescent microscope (ZEISS^{®} AXIOVERT^{®} A1, Carl Zeiss, Germany) equipped with 5X (EC PLAN-NEOFLUAR^{®}, NA 0.16) and 10X (EC PLAN-NEOFLUAR^{®}, NA 0.30) objectives, LED 385nm, LED 470nm and LED N-White, light sources and corresponding filter cubes for Blue, Green and Red emission detection (CARL ZEISS^{®} filter sets Nr.49, Nr.44 and 43). Images were processed and analyzed using CARL ZEISS^{®}, ZEN Blue v.2.6 and IMAGEJ^{™} software packages.

## Claims

1. A method of generating three-dimensional biological structures, the method comprising:
(a) depositing, using a flow-confinement device, a first layer of a suspension in a pattern over a substrate, the suspension comprising a liquid and a plurality of cells;
(b) allowing the plurality of cells to attach to the substrate and form a first layer of attached cells;
(b') culturing the plurality of cells in the first layer for a period of time of between about 10 minutes and about 8 weeks, in order to establish or increase the amount of one or more selected from the group consisting of: cell-cell interactions, cell-cell attachments, cell mass, and cell quantity, the culturing resulting in a tissue-like structure;
(c) depositing a cell-attachment agent over the first layer of attached cells;
(d) depositing, using a flow-confinement device, a second layer of the suspension in a pattern over the cell-attachment agent; and
(e) repeating steps (b)-(d) until a biological structure having a predefined thickness is generated,
where steps a)-d) are not performed on the human or animal body.

2. The method of claim 1, further comprising measuring protein expression, gene expression, metabolic markers, cell-cell interactions, ions, or other biological, physical or chemical properties of the plurality of cells, including recirculatory flow.

3. The method of claim 1, wherein the period of time comprises one or more ranges of time selected from the group consisting of: from about 10 minutes to about 30 minutes, from about 30 minutes to about 60 minutes, from about 1 hour to about 12 hours, form about 12 hours to about 1 day, from about 1 day to about 3 days, from about 3 days to about 1 week, from about 1 week to about 2 weeks, from about 2 weeks to about 4 weeks, and from about 4 weeks to about 8 weeks.

4. The method of claim 1, wherein the predefined thickness is selected from the group consisting of: about 1 µm, between about 1 µm and about 10 µm, between about 10 µm and about 100 µm, between about 100 µm and about 1 mm, between about 1 mm and about 1.5 mm, between about 1.5 mm and about 5 mm, between about 5 mm and about 2 cm, between about 2 cm and 10 cm, and between about 10 cm and 50 cm .

5. The method of claim 1, further comprising:
(e) repeating steps (a)-(c) at a new location on the substrate.

6. The method of claim 1, wherein the cell-attachment agent is applied in a pattern.

7. The method of claim 1, wherein the suspension comprises a plurality of cell types.

8. The method of claim 1, further comprising:
(f) depositing a cell blocking agent over the second layer of the suspension.

9. The method of claim 8, wherein the cell-blocking agent is selected from the group consisting of: polyethylene glycol (PEG), block copolymers, bovine serum albumin (BSA), surfactant, casein, polytetrafluoroethylene (PTFE), silanes, particulate solutions, nanoparticles, metal nanoparticles, gold nanoparticles, silver nanoparticles, aluminum nanoparticles, titanium nanoparticles, and polymeric microspheres.

10. The method of claim 1, wherein the cell-attachment agent is selected from the group consisting of: adhesion proteins, cell-attachment proteins, cell-adhesion biomolecules, cell-adhesion molecules, polylysine, laminin, fibronectin, vitronectin, extracellular matrix (ECM), actin, integrin, perlecan, desmin, poly glycans, fibulin, elastin, tropoelastin, cadherin, gelatin, concanavalin, collagen, lipid structures, lipid nanotubes, lipid vesicles, lipid droplets, growth serum, fetal bovine serum, calf serum, horse serum, nucleic acids, DNA strands, RNA strands, cationic solutions, calcium solutions, magnesium solutions, and zinc solutions.

11. The method of claim 1, wherein the substrate is selected from the group consisting of: cell-attachment agent, plastic, glass, silicon, paper, cotton, biomaterial, lipid films, gel, aerogel, hydrogel, polymer matrix, metals, metal oxides, semipermeable membranes, permeable membranes, polysaccharides, starch, collagen, cellulose, gelatin, biological scaffolds, biological tissue, decellularized tissue, non-biological tissue, and biological cells.

12. The method of claim 1, wherein the substrate comprises adjacent sections of different substrate materials.

## Patentansprüche

1. Verfahren zur Erzeugung dreidimensionaler biologischer Strukturen, wobei das Verfahren umfasst:
(a) Ablagerung einer ersten Schicht einer Suspension in einem Muster über einem Substrat unter Verwendung einer Vorrichtung zur Strömungsbegrenzung, wobei die Suspension eine Flüssigkeit und eine Vielzahl von Zellen umfasst;
(b) Ermöglichen, dass sich die Vielzahl von Zellen an das Substrat anlagert und eine erste Schicht von angelagerten Zellen bildet;
(b') Kultivieren der Vielzahl von Zellen in der ersten Schicht für eine Zeitspanne zwischen etwa 10 Minuten und etwa 8 Wochen, um die Menge von einem oder mehreren, ausgewählt aus der Gruppe bestehend aus Zell-Zell-Wechselwirkungen, Zell-Zell-Verbindungen, Zellmasse und Zellmenge, zu etablieren oder zu erhöhen, wobei die Kultivierung zu einer gewebeartigen Struktur führt;
(c) Aufbringen eines Zellanhaftungsmittels auf die erste Schicht von angehefteten Zellen;
(d) Ablagerung einer zweiten Schicht der Suspension in einem Muster über dem Zellanhaftungsmittel unter Verwendung einer Vorrichtung zur Strömungsbegrenzung; und
(e) Wiederholung der Schritte (b)-(d), bis eine biologische Struktur mit einer vordefinierten Dicke erzeugt ist,
wobei die Schritte a)-d) nicht am menschlichen oder tierischen Körper durchgeführt werden.

2. Verfahren nach Anspruch 1, das ferner umfasst: Messung der Proteinexpression, der Genexpression, der Stoffwechselmarker, der Zell-Zell-Wechselwirkungen, der Ionen oder anderer biologischer, physikalischer oder chemischer Eigenschaften der Vielzahl von Zellen, einschließlich der Rezirkulationsströmung.

3. Verfahren nach Anspruch 1, wobei die Zeitspanne einen oder mehrere Zeitbereiche umfasst, die ausgewählt sind aus der Gruppe bestehend aus: von etwa 10 Minuten bis etwa 30 Minuten, von etwa 30 Minuten bis etwa 60 Minuten, von etwa 1 Stunde bis etwa 12 Stunden, von etwa 12 Stunden bis etwa 1 Tag, von etwa 1 Tag bis etwa 3 Tage, von etwa 3 Tagen bis etwa 1 Woche, von etwa 1 Woche bis etwa 2 Wochen, von etwa 2 Wochen bis etwa 4 Wochen und von etwa 4 Wochen bis etwa 8 Wochen.

4. Verfahren nach Anspruch 1, wobei die vordefinierte Dicke ausgewählt ist aus der Gruppe bestehend aus: etwa 1 µm, zwischen etwa 1 µm und etwa 10 µm, zwischen etwa 10 µm und etwa 100 µm, zwischen etwa 100 µm und etwa 1 mm, zwischen etwa 1 mm und etwa 1,5 mm, zwischen etwa 1,5 mm und etwa 5 mm, zwischen etwa 5 mm und etwa 2 cm, zwischen etwa 2 cm und 10 cm, und zwischen etwa 10 cm und 50 cm.

5. das Verfahren nach Anspruch 1, das ferner umfasst:
(e) Wiederholung der Schritte (a)-(c) an einer neuen Stelle des Substrats.

6. Verfahren nach Anspruch 1, wobei das Zellanhaftungsmittel in einem Muster aufgetragen wird.

7. Verfahren nach Anspruch 1, wobei die Suspension eine Vielzahl von Zelltypen umfasst.

8. das Verfahren nach Anspruch 1, das ferner umfasst:
(f) Aufbringen eines Zellblockierungsmittels auf die zweite Schicht der Suspension.

9. Verfahren nach Anspruch 8, wobei das Zellblockierungsmittel ausgewählt ist aus der Gruppe bestehend aus: Polyethylenglykol (PEG), Blockcopolymeren, Rinderserumalbumin (BSA), Tensid, Kasein, Polytetrafluorethylen (PTFE), Silanen, partikulären Lösungen, Nanopartikeln, Metallnanopartikeln, Goldnanopartikeln, Silbernanopartikeln, Aluminiumnanopartikeln, Titannanopartikeln und polymeren Mikrosphären.

10. Verfahren nach Anspruch 1, wobei das Zellanhaftungsmittel ausgewählt ist aus der Gruppe bestehend aus: Adhäsionsproteinen, Zellanhaftungsproteinen, Zelladhäsions-Biomolekülen, Zelladhäsionsmolekülen, Polylysin, Laminin, Fibronektin, Vitronektin, extrazellulärer Matrix (ECM), Actin, Integrin, Perlecan, Desmin, Polyglykanen, Fibulin, Elastin, Tropoelastin, Cadherin, Gelatine, Concanavalin, Kollagen, Lipidstrukturen, Lipid-Nanoröhrchen, Lipidvesikel, Lipidtröpfchen, Wachstumsserum, fötales Rinderserum, Kälberserum, Pferdeserum, Nukleinsäuren, DNA-Strängen, RNA-Strängen, kationische Lösungen, Calciumlösungen, Magnesiumlösungen und Zinklösungen.

11. Verfahren nach Anspruch 1, wobei das Substrat ausgewählt ist aus der Gruppe bestehend aus: Zellanhaftungsmittel, Kunststoff, Glas, Silikon, Papier, Baumwolle, Biomaterial, Lipidfilmen, Gel, Aerogel, Hydrogel, Polymermatrix, Metallen, Metalloxiden, semipermeablen Membranen, permeablen Membranen, Polysacchariden, Stärke, Kollagen, Cellulose, Gelatine, biologischen Gerüsten, biologischem Gewebe, dezellularisiertem Gewebe, nicht-biologischem Gewebe und biologischen Zellen.

12. Das Verfahren nach Anspruch 1, wobei das Substrat benachbarte Abschnitte aus unterschiedlichen Substratmaterialien umfasst.

## Revendications

1. Procédé de génération de structures biologiques tridimensionnelles, le procédé comprenant :
(a) le dépôt, utilisant un dispositif de confinement de flux, d'une première couche d'une suspension selon un certain motif sur un substrat, la suspension comprenant un liquide et une pluralité de cellules ;
(b) le fait de laisser la pluralité de cellules s'attacher au substrat et former une première couche de cellules attachées ;
(b') la culture de la pluralité de cellules dans la première couche pendant une période de temps comprise entre environ 10 minutes et environ 8 semaines, afin que soit établie ou augmentée la quantité d'un ou plusieurs choisis dans le groupe constitué par : les interactions cellule-cellule, les attachements cellule-cellule, la masse cellulaire, et la quantité de cellules, la culture ayant pour résultat une structure analogue à un tissu ;
(c) le dépôt d'un agent d'attachement cellulaire sur la première couche de cellules attachées ;
(d) le dépôt, utilisant un dispositif de confinement de flux, d'une deuxième couche de la suspension selon un certain motif sur l'agent d'attachement cellulaire ; et
(e) la répétition des étapes (b) à (d) jusqu'à ce qu'une structure biologique ayant une épaisseur prédéfinie soit générée,
dans lequel les étapes a) à d) ne sont pas effectuées sur un corps humain ou animal.

2. Procédé selon la revendication 1, comprenant en outre la mesure de l'expression de protéines, de l'expression de gènes, de marqueurs métaboliques, d'interactions cellule-cellule, d'ions, ou d'autres propriétés biologiques, physiques ou chimiques de la pluralité de cellules, y compris le flux recirculatoire.

3. Procédé selon la revendication 1, dans lequel la période de temps comprend une ou plusieurs plages de temps choisies dans le groupe constitué par : d'environ 10 minutes à environ 30 minutes, d'environ 30 minutes à environ 60 minutes, d'environ 1 heure à environ 12 heures, d'environ 12 heures à environ 1 jour, d'environ 1 jour à environ 3 jours, d'environ 3 jours à environ 1 semaine, d'environ 1 semaine à environ 2 semaines, d'environ 2 semaines à environ 4 semaines, et d'environ 4 semaines à environ 8 semaines.

4. Procédé selon la revendication 1, dans lequel l'épaisseur prédéfinie est choisie dans le groupe constitué par : environ 1 µm, entre environ 1 µm et environ 10 µm, entre environ 10 µm et environ 100 µm, entre environ 100 µm et environ 1 mm, entre environ 1 mm et environ 1,5 mm, entre environ 1,5 mm et environ 5 mm, entre environ 5 mm et environ 2 cm, entre environ 2 cm et 10 cm, entre environ 10 cm et 50 cm.

5. Procédé selon la revendication 1, comprenant en outre :
(e) la répétition des étapes (a) à (c) en un nouvel emplacement sur le substrat.

6. Procédé selon la revendication 1, dans lequel l'agent d'attachement cellulaire est appliqué selon un certain motif.

7. Procédé selon la revendication 1, dans lequel la suspension comprend une pluralité de types cellulaires.

8. Procédé selon la revendication 1, comprenant en outre :
(f) le dépôt d'un agent bloqueur de cellules sur la deuxième couche de la suspension.

9. Procédé selon la revendication 8, dans lequel l'agent bloqueur de cellules est choisi dans le groupe constitué par : le polyéthylèneglycol (PEG), les copolymères séquencés, la sérumalbumine bovine (SAS), un tensioactif, la caséine, le polytétrafluoroéthylène (PTFE), les silanes, les solutions particulaires, les nanoparticules, les nanoparticules métalliques, les nanoparticules d'or, les nanoparticules d'argent, les nanoparticules d'aluminium, les nanoparticules de titane, et les microsphères polymères.

10. Procédé selon la revendication 1, dans lequel l'agent d'attachement cellulaire est choisi dans le groupe constitué par : les protéines d'adhésion, les protéines d'attachement cellulaire, les biomolécules d'adhésion cellulaire, les molécules d'adhésion cellulaire, la polylysine, la laminine, la fibronectine, la vitronectine, une matrice extracellulaire (ECM), l'actine, l'intégrine, le perlécan, la desmine, les polyglycanes, la fibuline, l'élastine, la tropoélastine, la cadhérine, la gélatine, la concanavaline, le collagène, les structures lipidiques, les nanotubes lipidiques, les vésicules lipidiques, les gouttelettes lipidiques, un sérum de croissance, le sérum de veau foetal, le sérum de veau, le sérum de cheval, les acides nucléiques, les brins d'ADN, les brins d'ARN, les solutions cationiques, les solutions de calcium, les solutions de magnésium, et les solutions de zinc.

11. Procédé selon la revendication 1, dans lequel le substrat est choisi dans le groupe constitué par : un agent d'attachement cellulaire, un plastique, un verre, le silicium, le papier, le coton, un biomatériau, les films lipidiques, un gel, un aérogel, un hydrogel, une matrice polymère, les métaux, les oxydes métalliques, les membranes semi-perméables, les membranes perméables, les polysaccharides, l'amidon, le collagène, la cellulose, la gélatine, les charpentes biologiques, un tissu biologique, un tissu décellularisé, un tissu non biologique, et les cellules biologiques.

12. Procédé selon la revendication 1, dans lequel le substrat comprend des sections adjacentes de différents matériaux de substrat.
